# EUROPEAN PATENT SPECIFICATION

(11) **EP 4 056 714 B1**
(45) Date of publication and mention of the grant of the patent: **06.03.2024**
(21) Application number: 22171854.7
(22) Date of filing: 24.03.2020
(51) Int. Cl.: C12Q 1/6806, G01N 33/569

(54) **DETECTION OF SARS-COV-2 IN A PLURALITY OF BIOLOGICAL SAMPLES**
NACHWEIS VON SARS-COV-2 IN MEHREREN BIOLOGISCHEN PROBEN
DÉTECTION DE SARS-COV-2 DANS UNE PLURALITÉ D'ÉCHANTILLONS BIOLOGIQUES

(43) Date of publication of application: 14.09.2022
(62) Divisional of application: 20165401.9
(73) Proprietor: DRK-Blutspendedienst Baden-Württemberg - Hessen gemeinnützige GmbH, 60528 Frankfurt am Main (DE); Johann-Wolfgang-Goethe-Universität Frankfurt am Main, 60323 Frankfurt am Main (DE)
(72) Inventor: Schmidt, Michael, 61350 Bad Homburg (DE); Seifried, Erhard, 60320 Frankfurt am Main (DE); Ciesek, Sandra, 60322 Frankfurt am Main (DE); Berger, Annemarie, 60386 Frankfurt am Main (DE)
(74) Representative: Witte, Weller & Partner Patentanwälte mbB

(56) References cited:
- RUDOLF HANEL ET AL: "Boosting test-efficiency by pooled testing strategies for SARS-CoV-2", ARXIV.ORG, CORNELL UNIVERSITY LIBRARY, 201 OLIN LIBRARY CORNELL UNIVERSITY ITHACA, NY 14853, 22 March 2020 (2020-03-22), XP081627062,
- VICTOR M CORMAN ET AL: "Detection of 2019 novel coronavirus (2019-nCoV) by real-time RT-PCR", EUROSURVEILLANCE, vol. 25, no. 3, 23 January 2020 (2020-01-23), XP055695049, FR ISSN: 1560-7917, DOI: 10.2807/1560-7917.ES.2020.25.3.2000045
- ROBERT DORFMAN: "The Detection of Defective Members of Large Populations", ANNALS OF MATHEMATICAL STATISTICS, INSTITUTE OF MATHEMATICAL STATISTICS, BALTIMORE, MD, US, vol. 14, no. 4, 1 December 1943 (1943-12-01), pages 436-440, XP009185271, ISSN: 0003-4851, DOI: 10.1214/AOMS/1177731363
- T. T. VAN ET AL: "Pooling Nasopharyngeal/Throat Swab Specimens To Increase Testing Capacity for Influenza Viruses by PCR", JOURNAL OF CLINICAL MICROBIOLOGY, vol. 50, no. 3, 28 December 2011 (2011-12-28), pages 891-896, XP055694732, US ISSN: 0095-1137, DOI: 10.1128/JCM.05631-11
- S. M. TAYLOR ET AL: "High-Throughput Pooling and Real-Time PCR-Based Strategy for Malaria Detection", JOURNAL OF CLINICAL MICROBIOLOGY, vol. 48, no. 2, 25 November 2009 (2009-11-25), pages 512-519, XP055694747, US ISSN: 0095-1137, DOI: 10.1128/JCM.01800-09
- M. J. CURRIE ET AL: "Pooling of Clinical Specimens Prior to Testing for Chlamydia trachomatis by PCR Is Accurate and Cost Saving", JOURNAL OF CLINICAL MICROBIOLOGY, vol. 42, no. 10, 1 October 2004 (2004-10-01), pages 4866-4867, XP055694749, US ISSN: 0095-1137, DOI: 10.1128/JCM.42.10.4866-4867.2004
- J C EMMANUEL ET AL: "Pooling of sera for human immunodeficiency virus (HIV) testing: an economical method for use in developing countries.", JOURNAL OF CLINICAL PATHOLOGY, vol. 41, no. 5, 1 May 1988 (1988-05-01), pages 582-585, XP055694750, GB ISSN: 0021-9746, DOI: 10.1136/jcp.41.5.582
- N N: "Pooling Method for Accelerated Testing of COVID-19", , 18 March 2020 (2020-03-18), XP055960159, Retrieved from the Internet: URL:https://www.technion.ac.il/en/2020/03/ pooling-method-for-accelerated-testing-of- covid-19/ [retrieved on 2022-09-12]

## Description

### Detection of SARS-CoV-2 in a plurality of biological samples

The present invention relates to a method for the detection of SARS-CoV-2 in a plurality of biological samples of living beings.

### FIELD OF THE INVENTION

The invention relates to the field of molecular biology, more particular to the detection of viral material in a biological sample.

### BACKGROUND OF THE INVENTION

At the end of the last century the HIV AIDS scandal [13,22,39] enables the integration of a new technology into blood donor screening by the investigation of molecular parameters by nucleic acid technologies (NAT) [1,6,11,16,29,31-34]. In the beginning only a few medical laboratories were able to implement this technology in the late 90ies into blood donor screening. For NAT it was strictly necessary to have separate rooms (pre-NAT room, NAT room and post NAT room). Staff were trained to work in an one-way-direction and it was strictly forbidden to go back to the NAT room after entry the post-NAT room on the same day. The detection of RNA and DNA viruses was a further challenge to screening tests. In the beginning samples tubes have to be open again after the reverse transcription step to change the enzyme from a reverse transcriptase into a DNA polymerase. But the technique developed rapidly and complete NAT robot systems are now available for blood donor screening [7,12,15] as well as for virus detection in clinical laboratories.

Current screening NAT robot systems (e.g. Roche Cobas 8800 or Grifols Panther) [14] have implemented different chambers to separate the pre-NAT room, from the NAT room and the post-NAT room. Working with these "all in one NAT systems" needs only trained staff, input of individual samples or mini-pool samples, reagents and disposables.

In 2019, a new Corona virus was first discovered in China named SARS-CoV-2. The new virus spread quickly from person to person. Due to intensive human travel activities, SARS-CoV-2 infected people worldwide. In March 2020 the WHO declared all criteria of a pandemic were fulfilled. In young people SARS-CoV-2 induce flu-like symptoms such as cough, sore throat, diarrhea or fever. However, severe pneumonia with fatalities also occurs in some cases especially in immunosuppressed people, old people or people with lung diseases like bronchial asthma. Up to now no suitable therapy is available. Therefore, the global community is relying on an early diagnosis with subsequent isolation of the infected people in order to slow down the further spread of SARS-CoV-2. As a result, since March 2020, many countries have introduced special measures as part of their national pandemic plans that restrict people's freedom of movement. Early diagnosis is possible using the nucleic acid amplification technique (NAT). This enables the detection of the virus directly. However, due to the exponentially increasing number of infected people, there is also a shortage of reagents with this detection method, so that not all people who are first contact persons to SARS-CoV-2 infected people or have mild flu-like symptoms can receive a NAT examination.
https://www.technion.ac.il/en/2020/03/pooling-method-for-accelerated-testing-of-covid-19/ suggests a pooling of probes to increase the through-put of SARS-CoV2 screening assays.

Against this background it is an object underlying the invention to provide a method for the detection of SARS-CoV-2 which overcomes the disadvantages in the art. Preferably such a method should be provided which allows acceleration of the test throughput and an increase of the test capacity over the current detection methods. Furthermore, such a detection method should be provided which requires less reagents, process steps, test equipment and utensils.

The present invention satisfies these and other needs.

### SUMMARY OF THE INVENTION

The present disclosure provides a method for the detection of SARS-CoV-2 in a plurality of biological samples of living beings comprising the following steps:
(1) Providing at least one biological sample S₁ of a first living being, said sample S₁ is suspected of containing SARS-CoV-2 and/or SARS-CoV-2 derived material;
(2) Providing at least one biological sample S₂ of at least a second living being, said sample is suspected of containing SARS-CoV-2 and/or SARS-CoV-2 derived material;
(3) Pooling at least an aliquot of said at least one biological sample S₁ and at least an aliquot of said at least one biological sample S₂ to obtain a pool sample S_{P};
(4) Testing the pool sample S_{P} for the presence of SARS-CoV-2 and/or SARS-CoV-2 derived material;
(5) Qualifying said first and said at least second living being as SARS-CoV-2 negative if in step (4) no SARS-CoV-2 and/or SARS-CoV-2 derived material is detected in pool sample S_{P};
   or
   Testing sample S₁ and sample S₂ individually for the presence of SARS-CoV-2 and/or SARS-CoV-2 derived material if in step (4) SARS-CoV-2 and/or SARS-CoV-2 derived material is detected in pool sample S_{P}.

Such method is disclosed but not claimed. By the simultaneous testing of at least two biological samples, i.e. biological samples from at least two living beings, e.g. humans or human patients, respectively, the performance of testing is significantly increased. Furthermore, the available test resources are employed in a more efficient manner by - in case of a negative outcome in step (4) - obtaining a test result for more than one living being in a single round of the method.

According to this method, "at least a second living being" means 1, 2, 3, 4, 5, 6, 7, 8, 9, 10, ... 49, ..., 99, ..., 199, ..., 299, ... 399, ... 499 etc. additional biological sample or additional living beings to be tested by the method according to the invention.

According to the invention "SARS-CoV-2 derived material" refers to structures, molecules or features being part of or originating from SARS-CoV-2 being suitable of indicating the presence of SARS-CoV-2 in the biological sample. "SARS-CoV-2 derived material" includes, e.g., nucleic acid, proteins, peptides and fragments thereof.

In this method said first and said at least second living being represent a group of living beings, said group consisting of a number of living beings which is selected from an integer of 2 - 500, preferably of 3 - 100, further preferably of 5 - 50, and further preferably of 10.

This measure has the advantage that by the method according to the invention biological samples of up to 500 living beings, e.g. humans or human patients, respectively, can be rather simultaneously tested in one round of the method.

In the method according to the invention said biological samples are swabs, preferably swabs from the throat.

This measure has the advantage that such kind of sample provision is employed which ensures, due to the high concentration of the virus in the throat area, that best possible biological source material in subjected to the method according to the invention. "Swab" is to be understood as referring to the wiped-off biological material itself and, alternatively, to the stick or cotton stick comprising the biological material.

In an embodiment of the method according to the invention said SARS-CoV-2 derived material is SARS-CoV-2 nucleic acid.

This measure has the advantage that the detection can be carried out via standard laboratory methods allowing the direct detection of the virus or material derived therefrom in a reliable manner.

In another embodiment of the method according to the invention said testing of the samples for the presence of SARS-CoV-2 and/or SARS-CoV-2 derived material is made by nucleic amplification technique (NAT), preferably by polymerase chain reaction (PCR).

This measure takes the advantage of using well-established and highly reliably detection methods, rendering the method according to the invention very accurate.

Another subject-matter according to the disclosure relates to a method for the detection of SARS-CoV-2 in a plurality of biological samples of living beings, preferably such a method as disclosed in the preceding paragraphs, said method comprises the following steps:
(1) Providing n biological samples, each thereof originates from an individual living being out of a group of *n* individual living beings, wherein *n* is an integer of 2 - 500, preferably of 3 - 100, further preferably of 5 - 50, and further preferably of 10;
(2) Placing each of said *n* biological samples in individual buffer solution and incubating for a time span allowing the displacement of SARS-CoV-2 and/or SARS-CoV-2 derived material from the biological sample and its transfer into the buffer to obtain individual primary samples S*ₙ*;
(3) Removing an aliquot from the individual primary samples S*ₙ* and diluting it in buffer by 1:*n* to obtain individual diluted samples S_{D*n*};
(4) Pooling said individual diluted samples S_{D*n*} to obtain a pool sample S_{P}.
(5) Testing the pool sample S_{P} for the presence of SARS-CoV-2 and/or SARS-CoV-2 derived material;
(6) Qualifying each of the *n* individual living beings as SARS-CoV-2 negative if in step (5) no SARS-CoV-2 and/or SARS-CoV-2 derived material is detected in pool sample S_{P};
   or
   Testing each of said individual primary samples S*ₙ* for the presence of SARS-CoV-2 and/or SARS-CoV-2 derived material if in step (5) SARS-CoV-2 and/or SARS-CoV-2 derived material is detected in pool sample S_{P}.

Such method, which is disclosed but not claimed, is also referred to as "dilution method". The first biological sample, e.g. a first swab from a first individual, is provided and placed into a tube of buffer (primary sample or tube) for a time sufficient to dispense SARS-CoV2 or material derived therefrom into the buffer. In a next step a small volume of the primary sample or tube is diluted and transferred into the pool tube or pool sample, respectively. For two biological samples or swabs/beings (n = 2) a dilution of 1:2 is used for other numbers of biological samples or swabs/beings other dilutions were used, i.e. n = 3 a dilution of 1:3, n = 4 a dilution of 1:4, n = 5 a dilution of 1:5 etc. is used.

This measure has the advantage that an optimum test volume for the subsequent testing step is achieved. This is important because the test apparatus, such as the PCR device has a certain maximum sample volume which must not be exceeded.

The features, elements, and advantages disclosed for the method at the outset apply to the "dilution method" likewise and vice versa.

In a variant of the "dilution method" in step (1) each of said n biological samples is provided via a swab, preferably a throat swab.

This measure has the advantage that such kind of sample provision is employed which ensures, due to the high concentration of the virus in the throat area, that best possible biological source material in subjected to the method according to the invention.

Another subject-matter of the disclosure relates to a method for the detection of SARS-CoV-2 in a plurality of biological samples of living beings, preferably such a method as disclosed in the preceding paragraphs, said method comprises the following steps:
(1) Providing 2*n* biological samples (double sample), each thereof originates from an individual living being out of a group of *n* individual living beings, wherein *n* is an integer of 2 - 500, preferably of 3 - 100, further preferably of 5 - 50, and further preferably of 10;
(2) Placing the first of said 2*n* biological samples (double sample) in individual buffer solution to obtain individual archive samples S_{A*n*};
(3) Pooling the second of said 2*n* biological samples (double sample) by placing them in common buffer solution and incubating them for a time span allowing the displacement of SARS-CoV-2 and/or SARS-CoV-2 derived material from the sample and its transfer into the common buffer to obtain a pool sample S_{P}.
(4) Testing the pool sample S_{P} for the presence of SARS-CoV-2 and/or SARS-CoV-2 derived material;
(5) Qualifying each of the *n* individual living beings as SARS-CoV-2 negative if in step (4) no SARS-CoV-2 and/or SARS-CoV-2 derived material is detected in pool sample S_{P};
   or
   Testing each of said individual archive sample S_{A*n*} for the presence of SARS-CoV-2 and/or SARS-CoV-2 derived material if in step (4) SARS-CoV-2 and/or SARS-CoV-2 derived material is detected in pool sample S_{P}.

Such method, which is disclosed but not claimed, is also referred to as "dual swab method". Providing "2n biological samples" means that per living being or individual out of n samples or individuals two samples or a "double sample" are provided, e.g. two throat swabs for each being are provided. One of the samples or swabs is placed into the archive sample or tube, respectively. The other sample or swamb is placed into the pool sample or tube. After the dispensing time the pool sample is analyzed.

The features, elements, and advantages disclosed for the method at the outset or the "dilution method" apply to the "dual swab method" likewise and vice versa.

In a variant of the "dual swab method" in step (1) each of said 2n biological samples (double samples) is provided via 2 swabs.

This measure has the advantage that such kind of sample provision is employed which ensures, due to the high concentration of the virus in the throat area, that best possible biological source material in subjected to the method according to the invention.

The invention relates to a method for the detection of SARS-CoV-2 in a plurality of biological samples of living beings, preferably such a method as disclosed in the preceding paragraphs, said method comprises the following steps:
(1) Providing *n* biological samples, each thereof originates from an individual living being out of a group of *n* individual living beings, wherein *n* is an integer of 2 - 500, preferably of 3 - 100, further preferably of 5 - 50, and further preferably of 10;
(2) Placing each of said *n* biological samples in individual buffer solution and incubating for a time span allowing the partial displacement of SARS-CoV-2 and/or SARS-CoV-2 derived material from the sample and its transfer into the buffer to obtain an individual archive sample S_{A*n*};
(3) Removing each of said *n* biological samples from the individual buffer, pooling them by placing them in common buffer solution and incubating them for a time span allowing the displacement of SARS-CoV-2 and/or SARS-CoV-2 derived material from the sample to obtain a pool sample S_{P}.
(4) Testing the pool sample S_{P} for the presence of SARS-CoV-2 and/or SARS-CoV-2 derived material;
(5) Qualifying each of the n individual living beings as SARS-CoV-2 negative if in step (4) no SARS-CoV-2 and/or SARS-CoV-2 derived material is detected in pool sample S_{P};
   or
   Testing each of said individual archive sample S_{A*n*} for the presence of SARS-CoV-2 and/or SARS-CoV-2 derived material if in step (4) SARS-CoV-2 and/or SARS-CoV-2 derived material is detected in pool sample S_{P}.

Such method is also referred to as "single swab method". The first or original sample or swab is placed first into an archive sample of tube, respectively. After the dispensing time which displaces a part of the virus or virus material from the sample or swab, however still leaves virus or virus material in the sample or swab sufficient for a later detection, the sample or swab is placed into the pool sample or tube. This procedure is repeated for further samples or swabs out of n samples or swabs, respectively.

The features, elements, and advantages disclosed for the method at the outset, the "dilution method" or the "dual swab method" apply to the "single swab method" likewise and vice versa.

In the "single swab method" in step (1) each of said n biological samples is a swab.

This measure has the advantage that such kind of sample provision is employed which ensures, due to the high concentration of the virus in the throat area, that best possible biological source material in subjected to the method according to the invention.

In an embodiment of the method according to the invention, in particular of the "single swab method" said incubation time span is in the range of approx. 1 second to approx. 2 hours.

Such measure has the advantage that such an incubation time is chosen which ensured the sufficient displacement of virus or virus material from the sample and its transfer into the buffer.

In an embodiment of the method according to the invention, in particular of the "single swab method" said buffer is conventional PCR medium.

By such measure a medium is used which allows a direct processing of the sample by conventional detection methods such as PCR.

Another subject-matter of the disclosure relates to a kit for the detection of SARS-CoV-2 in a plurality of biological samples of living beings, comprising swabs, test tubes, buffer solutions and a manual of the method according to the invention. This kit is disclosed but not claimed.

The features, characteristics, advantages and embodiments disclosed for the method according to the invention apply likewise to the kit according to the disclosure.

A "kit" is a combination of individual elements useful for carrying out the method of the invention, wherein the elements are optimized for use together in the methods. The kit may also contain additional reagents, chemicals, buffers, reaction vials etc. which may be useful for carrying out the method according to the invention. Such a kit unifies all essential elements required to work the method according to the invention, thus minimizing the risk of errors. Therefore, such kits also allow semi-skilled laboratory staff to perform the method according to the invention.

It is to be understood that the before-mentioned features and those to be mentioned in the following cannot only be used in the combination indicated in the respective case, but also in other combinations or in an isolated manner without departing from the scope of the invention.

The invention is now described and explained in further detail by referring to the following non-limiting examples and drawings.
- Figure 1:: Dilution of the original swab solution;
- Figure 2:: CT values for the individual detection as well as for a mini-pool of 2, a mini-pool of 3, a mini-pool of 5 and a mini-pool of 10 swabs;
- Figure 3:: Dual swab mini-pool method;
- Figure 4:: CT values for the dual swab method;
- Figure 5:: Single swab mini-pool method;
- Figure 6:: CT values for the single swab method.

### EMBODIMENTS

### Aims

The following three different independent embodiments of the methods according to the invention are illustrated which perform NAT detection in a mini-pool format in order to be able to examine more people without a significant reducing of the diagnostic NAT sensitivity.

The aim of the current validation was the comparison of an individual SARS-CoV-2 NAT detection with different mini-pool NAT formats.

### Results

In the first embodiment of the pooling method (dilution method, not claimed), the swabs are taken up in first step in a buffer and then different volumes are pipetted together depending on the pool size. The primary tubes in which the viruses were removed from the swab remain intact for a pool resolution. The validation results show comparable cycle threshold (CT) values up to a mini pool size of 3 (primary tube CT value of 24.33, mini-pool of 3 CT of 26.65).

The second embodiment (dual swab method, not claimed), 2 swabs were removed from each person. One swab is placed in the archive tube and the other swab is placed in the mini-pool tube. Since both swabs were removed at the same time, the virus concentration is comparable (CT value archive tube 24.87; CT value mini-pool tube 24.72). Comparable CT values between the swabs are shown in the validation. The method is also suitable for min-pool sizes between 2 and 100 samples.

The third embodiment (single swab method) is a method that works with a single swab. The swab is first placed in an archive tube which can be used for mini-pool deconstruction. After a suitable incubation period, the swab is transferred to the pool tube. In the validation, comparable CT values are shown between the archive tube (CT value 24.47) and the mini-pool tube (CT value 24.93). This method is suitable for mini-pool sizes between 2 and 100 samples.

### Conclusions

The three embodiments of pooling methods described are suitable for screening SARS CoV-2 in a mini-pool format to save limited resources of NAT reagents without losing significantly diagnostic NAT sensitivity. The Mini-Pool NAT procedure is particularly suitable for SARS-CoV-2 screening asymptomatic people who have little suspicion of a positive result.

### Material and Methods

### Swabs:

All conventional swabs are possible (e.g. Sarstedt forensic swab xl)

### Virus displacement solution:

All conventional reagents for virus displacement from the swabs are possible (e.g. Roche PCR media)

### Nucleic acid testing (NAT):

All NAT tests were performed on the Roche Cobas 6800/ 8800 instruments with the Roche SARS-CoV-2 assay.

### Sample collection

Due to the fact that SARS-CoV-2 infected primarily the nasopharynx area, samples were taken with dry swabs.

### Sample processing

In the next step the swabs were placed in a displacement solution (Guanidinium thiocyanate; GTC or PBS, or plasma, or other solutions). Then the RNA of SARS-CoV-2 is extracted manually of by robotic systems followed by a NAT method.

### First mini-pool format (dilution method, not claimed)

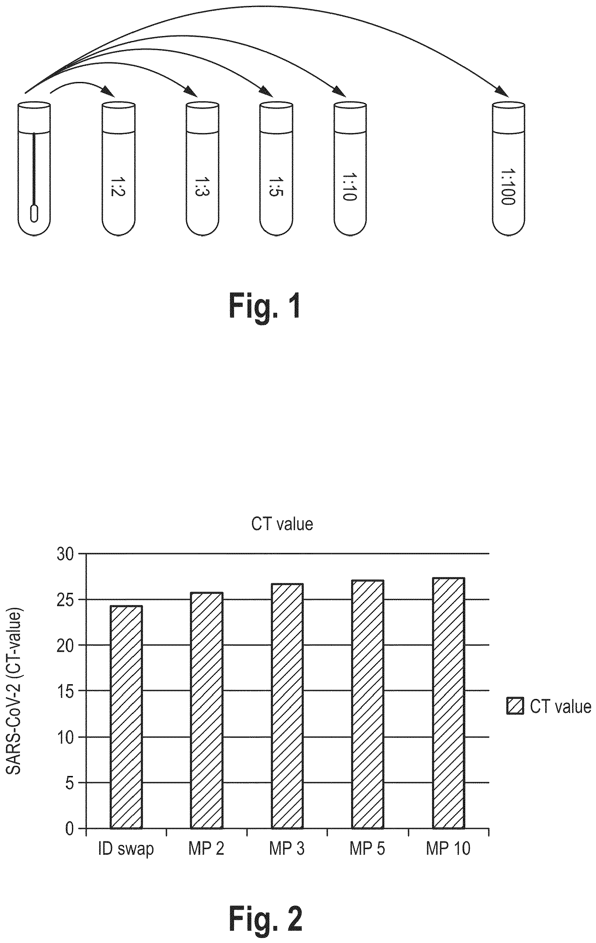

The original swab was placed into the displacement buffer for approximately 5 minutes to dispense SARS-CoV-2 from the swab into the buffer (all buffers are possible e. g. Roche PCR media). The dispensing time can be in a range between 1 second and 2 hours depending on the buffer used. In the next step a small volume of primary tube were transferred into the mini-pool tube. For two swabs mini-pools a dilution 1:2 is used. For other mini-pool formats other dilutions were taken (mini-pools of 3 a dilution 1:3; mini-pools of 4 a dilution 1:4; mini-pools of 5 a dilution 1:5). Figure 1 shows the dilution principle of this method.

Figure 2 shows the CT values for the individual detection as well as for a mini-pool of 2, a mini-pool of 3, a mini-pool of 5 and a mini-pool of 10 swabs.

The CT-value of individual swabs (24.33) were not significantly different to mini-pools of 2 swabs (25.82) or mini-pools of 3 swabs (26.65) and slightly increased in mini-pools of 5 (27.05) or mini-pools of 10 (27.40).

### Second mini-pool format (dual swab method, not claimed)

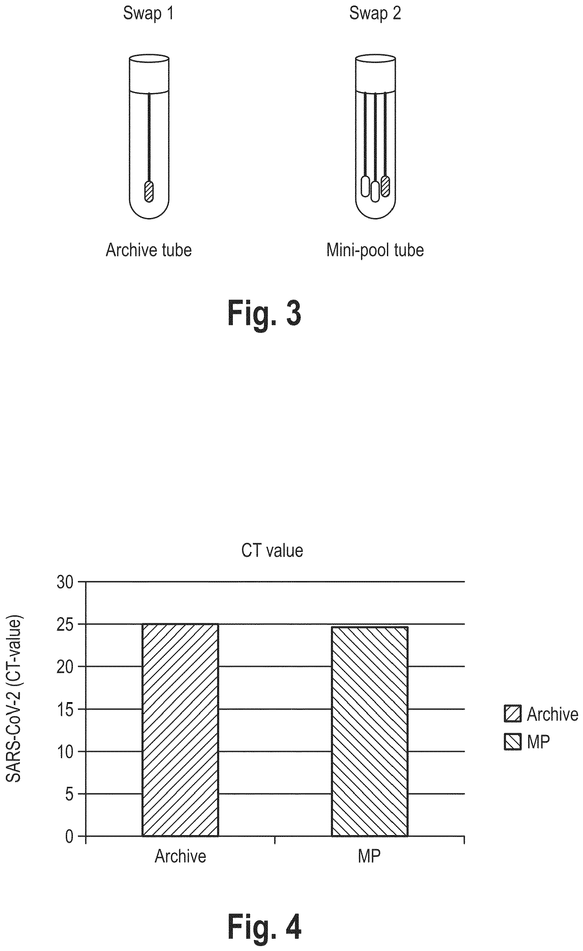

Two swabs were taken from each patient. One of the swabs was placed into the archive sample tube. The other swab was placed into a mini-pool tube. After the dispensing time the mini-pool tube was analyzed by the NAT system first. In case of a negative test result of the mini-pool test all sample included in the mini-pool are negative. In case of a positive mini-pool result, all archived samples of the mini-pool have to be investigated individually. Figure 3 shows the mini-pool procedure.

Figure 4 shows the CT values for archive swabs as well as for mini-pool swabs.

The CT-value of archive swabs (24.87) were not significantly different to mini-pools swabs (24.72). The dual swab mini-pool method is possible for mini-pools between 2 swabs and 100 swabs (preferably mini-pools of 5).

### Third mini-pool format (single swab method)

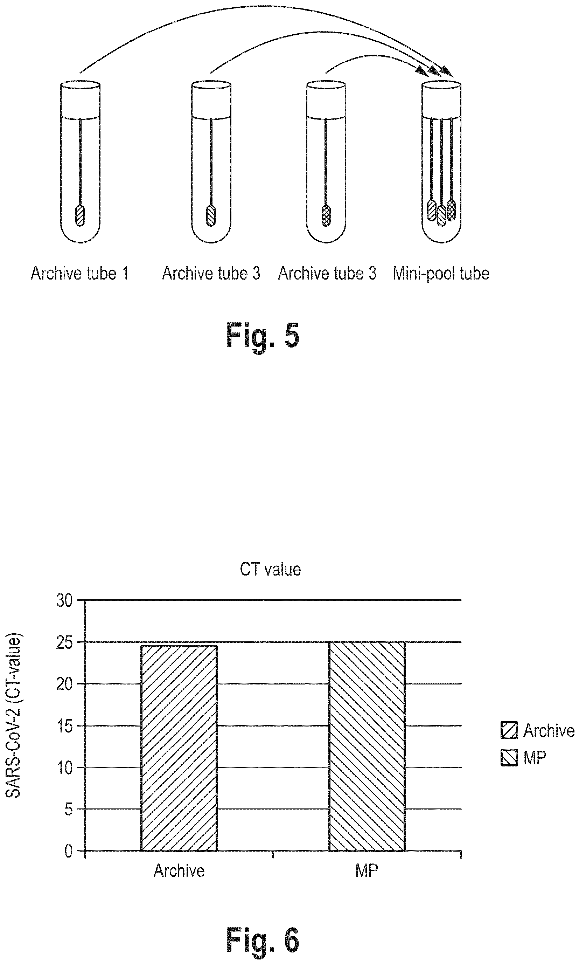

For the single swab method the original swab is placed first into an archive tube. After the dispensing time (range between 1 second and two hours, preferably 5 minutes) the swab was placed into the mini-pool tube starting a second dispensing time (range between 1 second and two hours, preferably 5 minutes). This procedure will be repeat for further swabs. In case of a negative test result of the mini-pool test all sample included in the mini-pool are negative. In case of a positive mini-pool result, all archived samples of the mini-pool have to be investigated individually. Figure 5 shows the mini-pool procedure.

Figure 6 shows the CT values for archive swabs as well as for mini-pool swabs.

The CT-value of archive swabs (24.47) were not significantly different to mini-pools swabs (24.93). The single swab mini-pool method is possible for mini-pools between 2 swabs and 100 swabs (preferably mini-pools of 5).

### Bottom line

In summary, pooling of swab samples for SARS-CoV-2 detection is possible as embodied by three different variants of methods without losing diagnostic sensitivity of the NAT detection method. The mini pool NAT procedure is required in a pandemic in order to optimally use of existing limited resources so that as many people as possible can benefit from the early detection of a SARS infection.

### References

1 Alter HJ, Houghton M: Clinical Medical Research Award. Hepatitis C virus and eliminating post-transfusion hepatitis. Nat Med 2000; 6:1082-1086.
2 Blumberg BS, Hesser JE, Economidou I, et al.: The variety of responses within a community to infection with Australia (hepatitis B) antigen. Dev Biol Stand 1975; 30:270-283.
3 Blumberg BS, Larouze B, London WT, et al.: The relation of infection with the hepatitis B agent to primary hepatic carcinoma. Am J Pathol 1975; 81:669-682.
4 Blumberg BS, London WT: Hepatitis B virus: pathogenesis and prevention of primary cancer of the liver. Cancer 1982; 50:2657-2665.
5 Busch MP: HIV, HBV and HCV: new developments related to transfusion safety. Vox Sang 2000; 78 Suppl 2:253-256.
6 Busch MP: Should HBV DNA NAT replace HBsAg and/or anti-HBc screening of blood donors? Transfus Clin Biol 2004; 11:26-32.
7 Busch MP, Sabino EC, Brambilla D, et al.: Duration of Dengue Viremia in Blood Donors and Relationships Between Donor Viremia, Infection Incidence and Clinical Case Reports During a Large Epidemic. J Infect Dis 2016; 214:49-54.
8 Chambers RW, Foley HT, Schmidt PJ: Transmission of syphilis by fresh blood components. Transfusion 1969; 9:32-34.
9 Cheng Y, Dubovoy N, Hayes-Rogers ME, et al.: Detection of IgM to hepatitis B core antigen in a reductant containing, chemiluminescence assay. J Immunol Methods 1999; 230:29-35.
10 Da Rin G, Zoppelletto M, Lippi G: Integration of Diagnostic Microbiology in a Model of Total Laboratory Automation. Lab Med 2016; 47:73-82.
11 Dodd R, Kurt Roth W, Ashford P, et al.: Transfusion medicine and safety. Biologicals 2009; 37:62-70.
12 Faddy HM, Flower RLP, Seed CR, et al.: Detection of emergent strains of West Nile virus with a blood screening assay. Transfusion 2016; 56:1503-1507.
13 Gedye R: German AIDS scandal infects Europe. BMJ 1993; 307:1229.
14 Grabarczyk P, Koppelman M, Boland F, et al.: Inclusion of human immunodeficiency virus Type 2 (HIV-2) in a multiplex transcription-mediated amplification assay does not affect detection of HIV-1 and hepatitis B and C virus genotypes: a multicenter performance evaluation study. Transfusion 2015; 55:2246-2255.
15 Grabarczyk P, van Drimmelen H, Kopacz A, et al.: Head-to-head comparison of two transcription-mediated amplification assay versions for detection of hepatitis B virus, hepatitis C virus, and human immunodeficiency virus Type 1 in blood donors. Transfusion 2013; 53:2512-2524.
16 Hourfar MK, Jork C, Schottstedt V, et al.: Experience of German Red Cross blood donor services with nucleic acid testing: results of screening more than 30 million blood donations for human immunodeficiency virus-1, hepatitis C virus, and hepatitis B virus. Transfusion 2008; 48:1558-1566.
17 Hourfar MK, Walch LA, Geusendam G, et al.: Sensitivity and specificity of Anti-HBc screening assays--which assay is best for blood donor screening? Int J Lab Hematol 2009; 31:649-656.
18 Joshi BN, Jundre S: Prevalence of Hepatitis B Antigen/antibody in Patients of Syphilis. Indian J Dermatol Venereol Leprol 1980; 46:335-337.
19 Khalil OS, Zurek TF, Tryba J, et al.: Abbott prism: a multichannel heterogeneous chemiluminescence immunoassay analyzer. Clin Chem 1991; 37:1540-1547.
20 Kleinman SH, Busch MP: HBV: amplified and back in the blood safety spotlight. Transfusion 2001; 41:1081-1085.
21 Kleinman SH, Kuhns MC, Todd DS, et al.: Frequency of HBV DNA detection in US blood donors testing positive for the presence of anti-HBc: implications for transfusion transmission and donor screening. Transfusion 2003; 43:696-704.
22 Kretschmer V: Infektionsrisiken von Blut und Blutprodukten im Zeichen des sogenannten AIDS-Skandals. Infusionsther Transfusionsmed 1993; 20:286-290.
23 Kuhns MC, Kleinman SH, McNamara AL, et al.: Lack of correlation between HBsAg and HBV DNA levels in blood donors who test positive for HBsAg and anti-HBc: implications for future HBV screening policy. Transfusion 2004; 44:1332-1339.
24 Lange W, Apodaca J, Kohler H: Antikorper gegen Hepatitis B (surface)-Antigen bei Hepatitispatienten und anderen epidemiologisch interessanten Bevolkerungsgruppen. Zentralbl Bakteriol Orig A 1975; 232:199-212.
25 Lange W, Kohler H, Apodaca J: Der Ausab-Test, ein Radioimmunoassay in der soliden Phase zum Nachweis von Antikorpern gegen Hepatitis B (Surface)-Antigen (Anti-HBsAg). Zentralbl Bakteriol Orig A 1974; 229:423-428.
26 Laperche S, Sauleda S, Piron M, et al.: Evaluation of the sensitivity and specificity performance of the Elecsys(R) HTLV-I/II assay in a multicenter study in Europe and Japan. J Clin Microbiol 2017.
27 Lelie N, Bruhn R, Busch M, et al.: Detection of different categories of hepatitis B virus (HBV) infection in a multi-regional study comparing the clinical sensitivity of hepatitis B surface antigen and HBV-DNA testing. Transfusion 2017; 57:24-35.
28 McCluskie JA: Transmission of Syphilis by Blood Transfusion. Br Med J 1939; 1:264-266.
29 Nubling CM, Heiden M, Chudy M, et al.: Experience of mandatory nucleic acid test (NAT) screening across all blood organizations in Germany: NAT yield versus breakthrough transmissions. Transfusion 2009; 49:1850-1858.
30 Roth WK, Busch MP, Schuller A, et al.: International survey on NAT testing of blood donations: expanding implementation and yield from 1999 to 2009. Vox Sang 2012; 102:82-90.
31 Roth WK, Seifried E: The German experience with NAT. Transfus Med 2002; 12:255-258.
32 Roth WK, Weber M, Buhr S, et al.: Yield of HCV and HIV-1 NAT after screening of 3.6 million blood donations in central Europe. Transfusion 2002; 42:862-868.
33 Schmidt M, Brixner V, Ruster B, et al.: NAT screening of blood donors for severe acute respiratory syndrome coronavirus can potentially prevent transfusion associated transmissions. Transfusion 2004; 44:470-475.
34 Schmidt M, Jimenez A, Muhlbacher A, et al.: Head-to-head comparison between two screening systems for HBsAG, anti-HBc, anti-HCV and HIV combination immunoassays in an international, multicentre evaluation study. Vox Sang 2015; 109:114-121.
35 Schryver A de, Meheus A: Syphilis and blood transfusion: a global perspective. Transfusion 1990; 30:844-847.
36 Schwarz HP, Dorner F: Karl Landsteiner and his major contributions to haematology. Br J Haematol 2003; 121:556-565.
37 Shimelis T, Lemma K, Ambachew H, et al.: Syphilis among people with HIV infection in southern Ethiopia: sero-prevalence and risk factors. BMC Infect Dis 2015; 15:189.
38 Wiener AS: Karl Landsteiner, M.D. History of Rh-Hr blood group system. N Y State J Med 1969; 69:2915-2935.
39 Zeiler T, Kretschmer V: Blutspenderbefragung zum Thema "Aufiniandsentschadigung fur Blutspender". Infusionsther Transfusionsmed 1995; 22:19-24.

## Claims

1. A method for the detection of SARS-CoV-2 in a plurality of biological samples of living beings, said method comprises the following steps:
(1) Providing *n* biological samples, each thereof originates from an individual living being out of a group of *n* individual living beings, wherein *n* is an integer of 2 - 500, preferably of 3 - 100, further preferably of 5 - 50, and further preferably of 10;
(2) Placing each of said *n* biological samples in individual buffer solution and incubating for a time span allowing the partial displacement of SARS-CoV-2 and/or SARS-CoV-2 derived material from the sample and its transfer into the buffer to obtain an individual archive sample S_{A*n*};
(3) Removing each of said n biological samples from the individual buffer, pooling them by placing them in common buffer solution and incubating them for a time span allowing the displacement of SARS-CoV-2 and/or SARS-CoV-2 derived material from the sample to obtain a pool sample S_{P}.
(4) Testing the pool sample S_{P} for the presence of SARS-CoV-2 and/or SARS-CoV-2 derived material;
(5) Qualifying each of the *n* individual living beings as SARS-CoV-2 negative if in step (4) no SARS-CoV-2 and/or SARS-CoV-2 derived material is detected in pool sample S_{P};
or
Testing each of said individual archive sample S_{A*n*} for the presence of SARS-CoV-2 and/or SARS-CoV-2 derived material if in step (4) SARS-CoV-2 and/or SARS-CoV-2 derived material is detected in pool sample S_{P}, wherein in step (1) each of said *n* biological samples is a swab.

2. The method of claim 1, wherein the swab is a swab from the throat.

3. The method of any of the preceding claims, wherein said SARS-CoV-2 derived material is SARS-CoV-2 nucleic acid.

4. The method of any of the preceding claims, wherein said testing of the samples for the presence of SARS-CoV-2 and/or SARS-CoV-2 derived material is made by nucleic amplification technique (NAT), preferably by polymerase chain reaction (PCR).

5. The method of any of the preceding claims, wherein said incubation time span is in the range of approx. 1 second to approx. 2 hours.

6. The method of any of the preceding claims, wherein said buffer is conventional PCR medium.

## Patentansprüche

1. Verfahren zur Detektion von SARS-CoV-2 in einer Vielzahl von biologischen Proben von Lebewesen, wobei das Verfahren die folgenden Schritte aufweist:
(1) Bereitstellen von *n* biologischen Proben, von denen jede von einem individuellen Lebewesen aus einer Gruppe von *n* individuellen Lebewesen stammt, wobei *n* eine ganze Zahl von 2 - 500, vorzugsweise von 3 - 100, weiter bevorzugt von 5 - 50 und noch weiter bevorzugt von 10 ist;
(2) Einbringen jeder der *n* biologischen Proben in eine individuelle Pufferlösung und Inkubieren für eine Zeitspanne, die die teilweise Ablösung von SARS-CoV-2 und/oder von SARS-CoV-2 abgeleitetem Material von der Probe und ihren Transfer in den Puffer ermöglicht, um eine individuelle Archivprobe S_{A*n*} zu erhalten;
(3) Entnehmen jeder der *n* biologischen Proben aus dem individuellen Puffer, Poolen derselben durch Einbringen in eine gemeinsame Pufferlösung und Inkubieren derselben für eine Zeitspanne, die die Ablösung von SARS-CoV-2 und/oder von SARS-CoV-2 abgeleitetem Material von der Probe ermöglicht, um eine Poolprobe S_{P} zu erhalten.
(4) Testen der Poolprobe S_{P} auf das Vorhandensein von SARS-CoV-2 und/oder von SARS-CoV-2 abgeleitetem Material;
(5) Qualifizierung jedes der *n* individuellen Lebewesen als SARS-CoV-2-negativ, wenn in Schritt (4) kein SARS-CoV-2 und/oder von SARS-CoV-2 abgeleitetes Material in der Poolprobe S_{P} detektiert wird;
oder
Testen jeder der individuellen Archivproben S_{A*n*} auf das Vorhandensein von SARS-CoV-2 und/oder von SARS-CoV-2 abgeleitetem Material, wenn in Schritt (4) SARS-CoV-2 und/oder von SARS-CoV-2 abgeleitetes Material in der Poolprobe S_{P} detektiert wird,
wobei in Schritt (1) jede der *n* biologischen Proben ein Abstrich ist.

2. Verfahren nach Anspruch 1, wobei es sich bei dem Abstrich um einen Abstrich aus dem Rachen handelt.

3. Verfahren nach einem der vorhergehenden Ansprüche, wobei das von SARS-CoV-2 abgeleitete Material SARS-CoV-2-Nukleinsäure ist.

4. Verfahren nach einem der vorhergehenden Ansprüche, wobei das Testen der Proben auf das Vorhandensein von SARS-CoV-2 und/oder von SARS-CoV-2 abgeleitetem Material durch Nukleinamplifikationstechnik (NAT), vorzugsweise durch Polymerase-Kettenreaktion (PCR), durchgeführt wird.

5. Verfahren nach einem der vorhergehenden Ansprüche, wobei die Inkubationszeitspanne im Bereich von ca. 1 Sekunde bis ca. 2 Stunden liegt.

6. Verfahren nach einem der vorhergehenden Ansprüche, wobei der Puffer ein herkömmliches PCR-Medium ist.

## Revendications

1. Procédé pour la détection du SARS-CoV-2 dans une pluralité d'échantillons biologiques d'êtres vivants, ledit procédé comprend les étapes suivantes :
(1) la fourniture de *n* échantillons biologiques, chacun d'entre eux provient d'un être vivant individuel parmi un groupe de *n* êtres vivants individuels, où *n* est un nombre entier allant de 2 à 500, de préférence de 3 à 100, en outre de préférence de 5 à 50, et en outre de préférence égal à 10 ;
(2) la mise de chacun desdits *n* échantillons biologiques dans une solution tampon individuelle et l'incubation pendant une durée permettant le déplacement partiel du matériel dérivé du SARS-CoV-2 et/ou du SARS-CoV-2 de l'échantillon et son transfert dans le tampon pour obtenir un échantillon d'archive individuel S*_{An}* ;
(3) le retrait de chacun desdits *n* échantillons biologiques du tampon individuel, leur regroupement en les mettant dans une solution tampon commune et en les incubant pendant une durée permettant le déplacement du matériel dérivé du SARS-CoV-2 et/ou du SARS-CoV-2 de l'échantillon pour obtenir un échantillon de groupe S_{P}.
(4) le test de l'échantillon de groupe S_{P} pour la présence de matériel dérivé du SARS-CoV-2 et/ou du SARS-CoV-2 ;
(5) la qualification de chacun des *n* êtres vivants individuels comme SARS-CoV-2 négatif si à l'étape (4) aucun matériel dérivé du SARS-CoV-2 et/ou SARS-CoV-2 n'est détecté dans l'échantillon de groupe S_{P} ;
ou
le test de chacun desdits échantillons d'archive individuels S*_{An}* pour la présence de matériel dérivé du SARS-CoV-2 et/ou du SARS-CoV-2 si à l'étape (4) le matériel dérivé du SARS-CoV-2 et/ou SARS-CoV-2 est détecté dans l'échantillon de groupe S_{P}, où à l'étape (1) chacun desdits *n* échantillons biologiques est un écouvillon.

2. Procédé de la revendication 1, dans lequel l'écouvillon est un écouvillon de la gorge.

3. Procédé de l'une des revendications précédentes, dans lequel ledit matériel dérivé du SARS-CoV-2 est un acide nucléique du SARS-CoV-2.

4. Procédé de l'une des revendications précédentes, dans lequel ledit test des échantillons pour la présence du matériel dérivé du SARS-CoV-2 et/ou du SARS-CoV-2 est effectué par une technique d'amplification nucléique (NAT), de préférence par une réaction en chaîne par polymérase (PCR).

5. Procédé de l'une des revendications précédentes, dans lequel ladite durée d'incubation se trouve dans l'intervalle allant d'environ 1 seconde à environ 2 heures.

6. Procédé de l'une des revendications précédentes, dans lequel ledit tampon est un milieu de PCR classique.
